(19) Europäisches Patentamt
European Patent Office
Office européen des brevets

(11) EP 1 338 269 B1

(12) **EUROPÄISCHE PATENTSCHRIFT**

(45) Veröffentlichungstag und Bekanntmachung des Hinweises auf die Patenterteilung:
**27.07.2005 Patentblatt 2005/30**

(51) Int Cl.7: **A61K 7/48**

(21) Anmeldenummer: **02003483.1**

(22) Anmeldetag: **15.02.2002**

(54) **Verwendung von Kreatinin und/oder Kreatinin-Derivaten zur Aufhellung der Haut und Milderung von Pigmentstörungen**

Use of creatinine and/or creatinine derivatives for whitening the skin and alleviating of pigmentation disorders

Utilisation de créatinine et/ou des dérivés de créatinine pour éclaircir la peau et pour soulager les désordres de pigmentation

(84) Benannte Vertragsstaaten:
**AT BE CH CY DE DK ES FI FR GB GR IE IT LI LU MC NL PT SE TR**

(43) Veröffentlichungstag der Anmeldung:
**27.08.2003 Patentblatt 2003/35**

(73) Patentinhaber: **Goldschmidt GmbH**
**45127 Essen (DE)**

(72) Erfinder:
- **Lersch, Peter, Dr.**
**46537 Dinslaken (DE)**
- **Weitemeyer, Christian, Dr.**
**45134 Essen (DE)**
- **Wollenweber, Ute**
**45130 Essen (DE)**

(56) Entgegenhaltungen:
**WO-A-00/15187** **US-A- 5 091 171**

- **PATENT ABSTRACTS OF JAPAN vol. 2000, no. 12, 3. Januar 2001 (2001-01-03) & JP 2000 247866 A (LION CORP), 12. September 2000 (2000-09-12)**

EP 1 338 269 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Gegenstand der Erfindung ist die Verwendung von Keratinin und/oder Kreatinin-Derivaten zur Aufhellung der Haut und Milderung von Pigmentstörungen.

Der Farbton der menschlichen Haut wird von der Menge des vorhandenen Melanins bestimmt. Melanin ist ein braun-schwarzes Pigment, welches in der Basalschicht der Epidermis von speziellen pigmentbildendenden Zellen, den Melanocyten gebildet wird. Diese Pigmentierung trägt zu einem Anteil am UV-Schutz der Haut bei, weil durch ihre Absorptionskapazität die schädliche UV-Strahlung stark abgeschwächt werden kann. Menschen unterschiedlicher Hautfarbe verfügen interessanterweise über eine vergleichbare Anzahl von Melanocyten, nur die Neubildungsgeschwindigkeit des Melanins, dessen Konzentration und Verteilung sind unterschiedlich.

UV-Bestrahlung induziert die Bildung von Melanin in speziellen Teilen der Melanocyten, den sogenannten Melanosomen. Das gebildete Melanin wird in die Keratinocyten transportiert und wird dort als braune Hautfarbe sichtbar. Je mehr Melanin produziert wird, desto dunkler und brauner ist die Haut.

Eine ungleiche Verteilung der Melanocyten in den Hautgewebepartien führt zum unerwünschten Auftreten von unterschiedlichen Hauttönen und lokalen unregelmäßigen Hyperpigmentierungen, welche sich z.B. in der Form von Schwangerschaftsflecken, Altersflecken, Sommersprossen oder anderen Pigmentstörungen äußern.

Chemisch handelt es sich bei Melanin um polymere Indol-5,6-Chinoide, welche in einer komplexen Reaktionskaskade aus der aromatischen Aminosäure L-Tyrosin gebildet werden. Der Reaktionsmechanismus wurde von Raper und Mason aufgeklärt und involviert Tyrosinasen als Klasse von Schlüsselenzymen.

Tyrosinase gehört zur Familie der Typ-3-Kupfer-Proteine und ist verantwortlich für die Hydroxylierung von Mono- zu Ortho-Diphenolen. Eine weitergehende Beschreibung findet sich in Angew. Chem. 2000, 112 (9), 1656-1660. Tyrosinase wird durch UV-Licht aktiviert, wodurch es die oxidative Umwandlung von L-Tyrosin zu L-3,4-Dihydroxyphenylalanin (L-Dopa) katalysiert. L-Dopa wiederum wird ebenfalls durch Tyrosinase in weiteren Reaktionsschritten zu Dopachinon und letztlich zu Melanin aufoxidiert. Dieser Mechanismus kommt ubiquitär in der Natur vor, so sind Tyrosinasen aus Pilzen, Pflanzen und Säugetieren bezüglich ihrer Substratspezifität und Wirkung direkt vergleichbar.

Unerwünschte Pigmentstörungen können mit Depigmentierungsmitteln behandelt werden, worunter man Präparate zur Hautbleichung bzw. Hautaufhellung versteht.

Insbesondere in den asiatischen Ländern haben viele Menschen das Bedürfnis ihre natürliche Hautfarbe aufzuhellen, da dies den dort vorherrschenden Schönheitsidealen entspricht.

In den westlichen Ländern besteht ein vermehrtes Interesse daran, das oftmals altersbedingte Auftreten unregelmäßig pigmentierter Hautpartien wie z.B. Schwangerschaftsflecken oder Altersflecken wirksam auszugleichen.

An Versuchen Pigmentstörungen zu beheben hat es bisher nicht gemangelt und es wurden in der Vergangenheit bereits eine Vielzahl unterschiedlicher Substanzen vorgeschlagen, welche ihrerseits in verschiedene Regulationsmechanismen der Pigmentbildung eingreifen.

Auf Hauttönungen und -störungen kann man gezielten Einfluss nehmen, indem entweder das vorhandene Melanin abgebaut wird oder eine Verringerung der Melaninbildung erreicht wird.

So wurden früher u.a. Quecksilber- und Wismutsalze verwendet, welche eine irreversible Hemmung der Tyrosinase bewirken. Wegen ihrer hohen Toxizität werden solche Substanzen jedoch heutzutage nicht mehr in kosmetischen Mitteln eingesetzt. Auch die Verwendung von zelltoxischen Verbindungen, wie Hydrochinon und dessen Derivate, welche eine direkte Zerstörung der Melanocyten hervorrufen und wegen ihrer hautschädigenden Wirkung nur auf kleinen Hautarealen angewendet werden können, sind in den meisten Ländern bereits nicht mehr zugelassen.

Handelsübliche Hautaufhellungsmittel enthalten daher in der Regel zumeist mehr oder weniger wirksame Tyrosinase-Inhibitoren. Eine Reihe von Substanzen hat diese Eigenschaften. Die Palette der verwendeten Materialien beinhaltet daher neben den unterschiedlichsten Pflanzenextrakten auch Vitamin C und Ascorbinsäurederivate sowie heterocyclische Verbindungen wie etwa Pyranonderivate.

Ein Überblick der Thematik und der verwendeten Substanzen findet sich u.a. in Cosmetics & Toiletries 1995, 110 (10), 51-56. Diese Substanzen werden bei genauer Betrachtung den an sie gestellten Ansprüchen jedoch nicht völlig gerecht.

In diesem Zusammenhang ist beispielsweise der Einsatz von Kojisäure (5-Hydroxy-2-hydroxymethyl-4-pyranon) bekannt. So werden in der GB-A-826244 Methoden zur fermentativen Herstellung von Kojisäure beschrieben, welche in einem mehrstufigen Verfahren aus Aspergilluskulturen gewonnen werden.

Beispiele für den Einsatz der Substanz und ihrer Derivate in Hautaufhellungsmitteln finden sich vielfach in der Patentliteratur. Stellvertretend hierfür sei auf die EP-A-0 308 543 hingewiesen.

Der Wirkmechanismus der Kojisäure und ihrer Derivate soll auf einer Chelatisierung des katalytisch aktiven Kupfer-Zentralatoms in der Tyrosinase beruhen. Für die Einarbeitung in kosmetische Präparate erweist sich jedoch die Unbeständigkeit der Kojisäure in wässrigen Lösungen als ein ziemlicher Nachteil, da sie neben einem hohen Wirksamkeitsverlust auch zu Stabilitätsproblemen der Formulierung beitragen kann.

Es besteht daher weiterhin ein Bedarf an Wirkstoffen für kosmetische und dermatologische Formulierungen,

die in der Lage sind, die natürliche Hautfarbe zu bleichen oder aufzuhellen und/oder das Auftreten unregelmäßig pigmentierter Hautpartien wie z.B. Schwangerschaftsflecken oder Altersflecken wirksam auszugleichen.

Vorzugsweise soll ein solcher Wirkstoff bereits in geringen Einsatzkonzentrationen eine deutliche Wirkung hervorrufen, nicht toxisch sein, vorzugsweise natürlichen Ursprungs sein, sehr gut von der Haut toleriert werden, eine hohe Verträglichkeit mit anderen Inhaltsstoffen aufweisen und sich problemlos in Hautbehandlungsmittel einarbeiten lassen.

Besonders wünschenswert ist es, wenn dieser Wirkstoff zusätzlich auch einfach und kostengünstig hergestellt werden kann und in einer Form anfällt, welche einfach aufgereinigt werden kann und damit die an kosmetische bzw. dermatologische Wirkstoffe gestellten hohen Reinheitsbedingungen erfüllt.

Es ist daher eine Aufgabe der Erfindung, derartige Wirkstoffe zur Verfügung zu stellen, welche in der Lage sind, in kosmetischen und dermatologischen Formulierungen die natürliche Hautfarbe aufzuhellen und/oder das Auftreten unregelmäßig pigmentierter Hautpartien wie z.B. Schwangerschaftsflecken oder Altersflecken wirksam auszugleichen.

Ein Gegenstand der JP 2000-247866 sind Hautkosmetika, welche Hautaufheller (whitening agents) wie Ellaginsäure, Hydrochinon, Arbutin, Kojisäure und anderen Materialien in Kombination mit Kreatin und/oder Kreatinin beinhalten und einen verbesserten Aufhellungseffekt aufweisen sollen.

In den Vergleichsbeispielen 1-9 (Tabellen 4 und 5) wird in der gleichen Basisrezeptur der Effekt der Aufheller (Wirkstoffe) allein, der Basisrezeptur allein, Kreatinin allein und die Kombination aus Aufheller und Kreatinin untersucht, mit dem Resultat dass Kreatinin allein keine aufhellende Wirkung hat, in Kombination mit aufhellend wirkenden Verbindungen jedoch eine Verstärkung der aufhellenden Wirkung erzielt werden kann.

Es wurde nun überraschenderweise gefunden, dass Kreatinin und/oder Kreatinin-Derivate in Zubereitungen zur Behandlung und Nachbehandlung der Haut alle diese gewünschten Kriterien erfüllen.

Ein Gegenstand der Erfindung ist daher die Verwendung von Verbindungen der allgemeinen Formel (I) und/oder deren Salze

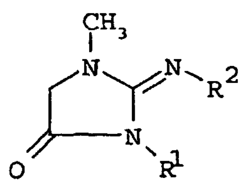

(I)

in denen

$R^1$ H, Alkyl-, Hydroxyalkyl-, Carboxyalkylrest mit 2 bis 30 C-Atomen;

$R^2$ H oder ein gegebenenfalls verzweigter, gegebenenfalls Doppelbindungen enthaltender Kohlenwasserstoffrest mit 1 bis 30 C-Atomen sein kann,

zur Aufhellung der Haut und Milderung von Pigmentstörungen

Ein weiterer Gegenstand der Erfindung ist die Verwendung von wässrigen kosmetischen oder dermatologischen Formulierungen enthaltend

a) 0,05 bis 10 Gew.-Teile mindestens einer der Verbindungen der allgemeinen Formel (I) und

b) 1 bis 10 Gew.-Teile mindestens eines Tensids aus der Gruppe der nichtionischen, amphoteren, zwitterionischen, ionischen Tenside und gegebenenfalls

c) 2 bis 10 Gew.-Teile pflanzliche oder mineralische Öle, Esteröle

d) 1 bis 5 Gew.-Teile Konsistenzgeber

e) 0,5 bis 5,0 Gew.-Teile Duftstoffe, Farbstoffe, Pflanzenextrakte, Konservierungsmittel

f) ad 100 Wasser

zur Aufhellung der Haut und Milderung von Pigmentstörungen.

Weitere Gegenstände der Erfindung werden durch die Ansprüche gekennzeichnet.

Kreatinin (CAS-Nr. 60-27-5) ist der Trivialname für das 2-Imino-N-methylhydantoin, ein zyklisches Kondensationsprodukt, welches durch intramolekulare Wasserabspaltung aus Kreatin erhalten werden kann (Formel II).

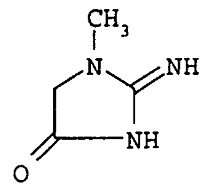

(II)

Kreatinin kommt natürlich im Körper vor und wird in der Medizin als ein biologischer Marker verwendet. Weiterführende Angaben hierzu finden sich u.a. bei D.W. Cockcroft und M.H.Gault in Prediction of creatinine clearance from serum creatinine, *Nephron*. 1976 (16)31-41.

Kreatinin findet sich regelmäßig im Urin und entsteht durch Übertragung der Amidinogruppierung von Arginin auf Glycin. Es ist auch im Schweiß enthalten und eine weitere Rolle kommt der Substanz als Bestandteil des natürlichen Feuchthaltefaktors der Haut zu.

In jüngerer Zeit wurden bereits einige wenige Patentanmeldungen veröffentlicht, in denen die Verwendung von Kreatinin in Hautpflegeprodukten beschrieben wird.

So ist aus der WO-A-00/15187 (SKW Trostberg) die Verwendung von Kreatin als Feuchthaltemittel in kosmetischen Zubereitungen bekannt. Der Einsatz von Kreatin und/oder geeigneter Derivate soll Symptome einer trokkenen Haut wie Risse und Schuppung nachhaltig beseitigen.

In der JP 2000-247866 (Lion Corporation) werden Hautkosmetika beschrieben, welche Kreatin und/oder Kreatinin in Verbindung mit einem weiteren pharmazeutischen Wirkstoff und/oder einer bioaktiven Substanz beinhalten und einen verbesserten Pflegeeffekt aufweisen sollen. Im Rahmen einer allgemeinen Aufzählung ist dort zwar die Verwendung von Kreatinin in Verbindung mit Hautaufhellern (whitening agents) wie Ellaginsäure, Hydrochinon, Arbutin, Kojisäure und anderen Materialien beschrieben. Die effektive hautaufhellende Wirkung wird hier allein auf diese Aufheller zurückgeführt. Die Wirkung von Kreatinin und seiner Derivate allein ist in dieser Veröffentlichung jedoch nicht erkannt worden und daher sind dieser Schrift auch keine Hinweis auf derartige Eigenschaften zu entnehmen.

Eine weitere Anmeldung ist die WO-A-01/00203 (Avicena), welche die Verwendung von Kreatin und Kreatinderivaten als Antioxidantien und zur Regeneration von gestresster Haut beansprucht.

Der Literatur lässt sich daher nichts über die Verwendung von Kreatinin oder dessen Derivate in Hautbehandlungsmitteln zum Zwecke der Hautaufhellung oder der Bleichung unregelmäßig pigmentierter Hautpartien entnehmen.

Als besonders geeignet im Sinne der vorliegenden Erfindung hat sich Kreatinin erwiesen und wird daher bevorzugt. Aber auch Kreatininderivate wie dessen Salze mit anorganischen Säuren wie beispielsweise Phosphorsäure, vorzugsweise organischen ein- oder mehrbasischen Säuren wie beispielsweise Essigsäure, Glycolsäure, Milchsäure, Zitronensäure, Apfelsäure, Salicylsäure oder Sorbinsäure und deren Gemische, oder wie Kreatininpyruvat sind sehr gut geeignet. Dabei ist es im Sinne der vorliegenden Erfindung auch möglich, geeignete Kreatininderivate untereinander in Mischungen zu verwenden.

Die Kreatininderivate der allgemeinen Formel (I) können nach üblichen Veresterungs-, Amidierungs- und Alkylierungs- bzw. Additionsverfahren oder in Anlehnung an diese Verfahren hergestellt werden. Hierzu sei auf die gängige Fachliteratur wie z.B. Houben-Weyl, Methoden der Organischen Chemie 4TH EDITION, Supplementary Series, Bände E4 CARBONIC ACID DERIVATIVES (1983) und E5 CARBOXYLIC ACID, DERIVATIVES (1985) sowie auf die jährlich erscheinenden Schriften zu "Guanidino Compounds in Biology and Medicine" (Eds. P.P. DeDeyn, B. Marescau, V. Stalon, J.A. Qureshi), John Libbey and Co. Ltd., London verwiesen.

Kreatinin selbst liegt in wässriger Lösung im Gleichgewicht mit Kreatin vor, wobei das Gleichgewicht in Abhängigkeit von ph-Wert und Temperatur verschoben werden kann. Es können daher für die kosmetischen oder dermatologischen Formulierungen sowohl Kreatin und Kreatin-/Kreatininmischungen verwendet werden, sofern durch Einhaltung entsprechender Lager- und/oder Anwendungsbedingungen sichergestellt ist, dass bei der Applikation ein ausreichend wirksamer Gehalt an Kreatinin vorhanden ist.

Als erfindungsgemäße, kosmetische oder dermatologische Zubereitungen zur Depigmentierung werden vor allem solche Mittel verstanden, die auf die Gesichtshaut und/oder andere hyperpigmentierte Körperpartien aufgetragen werden.

Diese Mittel sind die üblicherweiser verwendeten kosmetischen oder dermatologischen Formulierungen, die im Allgemeinen als wässrige alkoholische Lösungen, Cremes, Emulsionen, Lotionen, Gele, Ärosolspray oder -schaum, Nonärosolspray oder -schaum vorliegen und in denen die Verbindungen der allgemeinen Formel (I) mitverwendet werden und sie können demnach zur Anpassung auch weitere übliche Bestandteile enthalten, welche zur Behandlung,

Pflege, Reinigung und dem Schutz der Haut dienen, wie beispielsweise hautkosmetischen Wirkstoffen (active ingredients) wie z.B. Kreatin, Ceramide, Pseudoceramide, Proteinhydrolysate pflanzlichen oder tierischen Ursprungs auf Basis Keratin, Collagen, Elastin, Weizen, Reis, Soja, Milch, Seide, Mais, Aminosäuren und Aminosäurederivate, Polyasparaginsäure(derivate), entzündungshemmende Wirkstoffe, antimikriobelle Wirkstoffe, gebräuchliche Antioxidantien, Vitamine, Dexpanthenol, Milchsäure, Pyrrolidoncarbonsäure, Bisabolol sowie Pflanzen-, Hefe-, und Algenextrakten.

Die Kombination mit üblicherweise verwendeten organischen oder anorganischen UV-Filtersubstanzen ist als besonders vorteilhaft anzusehen, da während der Anwendung der Hautbleichungsformulierungen zum einen die Melaninneubildung der Haut wirksam unterbunden wird und dadurch die natürliche Schutzfunktion des Melanins reduziert wird, andererseits durch die Vermeidung von Sonnenlichteinstrahlung auch der Neuproduktion von Melanin vorgebeugt wird.

Zusätzlich können noch weitere kosmetische Hilfs- und Zusatzstoffe, die in solchen Zubereitungen üblich sind, enthalten sein. Solche Hilfstoffe sind z.B. Lösungsvermittler wie Ethanol, Isopropanol, Ehtylenglykol; Propylenglykol, Glycerin und Diethylenglykol. Zu den weiteren Komponenten zählen kosmetische Öle pflanzlichen und synthetischen Ursprungs, Emollients, Fette, Wachse, Rückfetter, Emulgatoren, Verdickungsmittel, anionische, zwitterionische, ampholytische und nichtionische Tenside sowie Duft- und Konservierungsstoffe.

Schließlich können die erfindungsgemäßen Formulierungen auch noch Komplexbildner wie EDTA, NTA, β-Alanindiessigsäure und Phosphonsäure, Farbstoffe zum Anfärben der kosmetischen Zubereitung, Trübungsmittel wie Latex, Styrol/PVP-und Styrol-Acrylamid-Copolymere, Perlglanzmittel wie Ethylenglykolmono- und -distearat und PEG-3-distearat, Pigmente, Lichtschutzmittel, Verdickungsmittel oder Treibmittel enthalten.

Typische Rahmenrezepturen für Hautbehandlungsmittel gehören zum bekannten Stand der Technik und sind beispielsweise in den Broschüren der Hersteller der jeweiligen Grund- und Wirkstoffe enthalten. Eine informative Quelle für derartige Formulierungen stellt z.B. das jährlich erscheinende Kosmetik-Jahrbuch (Herausgeber: B. Ziolkowsky, Verlag für Chemische Industrie) dar.

Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Kreatinin und dessen Derivate können dabei generell in einer Konzentration von 0,05 bis 10,0 Gew.-%, bevorzugt in einer Konzentration von 0,2 bis 5,0 Gew.-%, enthalten sein.

Ein Beispiel für eine durchaus übliche Rezeptur einer Hautcreme-Formulierung ist nachstehend angeführt. Diese setzt sich zusammen aus:

| Grundrezeptur: Hautcreme | | |
|---|---|---|
| Erfindungsgemäße Verbindung | 0,05 bis 10 | Gew.-Teile |
| Glycerinmonodistearat | 2 bis 10 | Gew.-Teile |
| Cetylalkohol | 1 bis 4 | Gew.-Teile |
| Paraffinöl 3,5°E | 4 bis 12 | Gew.-Teile |
| Glycerin | 1 bis 5 | Gew.-Teile |
| demin. Wasser | ad 100 | |
| Konservierungsmittel | n. B. | |

Die erfindungsgemäßen kosmetischen Zubereitungen zur aufhellenden Behandlung der Haut weisen einen pH-Wert von 3 bis 7 auf und enthalten daher bevorzugt eine dafür geeignete wasserlösliche Säure oder Puffergemische, welche diesen pH-Wert stabilisieren. Geeignete Säuren sind insbesondere die niedermolekularen organischen Säuren, wie beispielsweise Essigsäure, Glycolsäure, Milchsäure, Zitronensäure, Apfelsäure, Salicylsäure oder Sorbinsäure und Gemische dieser Säuren mit ihren Alkalisalzen.

Die Herstellung der erfindungsgemäßen Formulierungen erfolgt in der üblichen Weise wobei das Kreatin und dessen Derivate bevorzugt in der wässrigen Phase der Formulierung gelöst werden. Die Einstellung des pH-Wertes erfolgt bevorzugt zuletzt durch Zugabe der dafür vorgesehenen Säure und/oder des Puffergemisches. Zur besseren Löslichkeit des Kreatins und seiner Derivate kann die erfindungsgemäße Zubereitung vor der Anwendung auf dem Haar leicht erwärmt werden.

Die folgenden Beispiele sollen den Gegenstand der Erfindung näher erläutern:

Beispiel 1:

Tyrosinase-Hemmung durch Kreatinine

Die Inhibition von Pilz-Tyrosinase wurde in Abhängigkeit verschiedener Konzentrationen von Kreatinin (0,05 bis 1,7 Vol.-%) anhand der enzymatischen Umsetzung von L-DOPA zu Dopachrom bestimmt. Das Absorptionsmaximum des Dopachroms liegt bei einer Wellenlänge von 475 nm. Die Tyrosinase-Inhibition errechnet sich nach folgender Gleichung:

$$(1) \qquad \text{Inhibition [\%]} = \{100 - [(E_{Probe} *100) / E_{Referenz}]\}$$

Jede Messung wurde zweifach, in parallelen Ansätzen durchgeführt. Die Schwankung der Methode liegt bei ± 10 %.

Verwendete Chemikalien:

L-3.4-Dihydroxyphenylalanin (L-DOPA) (Sigma)
Tyrosinase, 25.000 units (Sigma)

Verwendete Lösungen:

25 mM Acetat-Puffer (pH 4,9): Verdünnen von 0,2 M Acetat-Puffer (pH 4,9) (Sigma)
Phosphat-Puffer (pH 7): Titrisol (Merck)
20 mM L-DOPA (in Acetat-Puffer)
Tyrosinase-Stammlösung: 40 U/ mL Phosphat-Puffer
Stammlösung Kreatinin: 0,45 M in Phosphat-Puffer

Die DOPA-Lösung sowie die Tyrosinase-Stammlösung wurden erst vor Versuchsbeginn hergestellt. Die L-DOPA-Lösungen sollten dunkel und in gut verschlossenen Gefäßen aufbewahrt werden.

Durchführung:

500 µL DOPA-Stammlösung
400 µL Kreatinin-Lösung bzw. Phosphat-Puffer (Referenz)
100 µL Tyrosinase-Stammlösung

Nach Zugabe des Enzyms wurden die Proben am "Reax Top" (Fa. Heidolph) gut durchgemischt. Nach einer Inkubation von 15 Min. bei Raumtemperatur wurde die Extinktion bei 475 nm detektiert. Die Messung erfolgte mit einem "Helios Beta"-Spektralfotometer der Firma Unicam.

Ergebnis:

In Tabelle 1 ist die Tyrosinase-Inhibition in Abhängigkeit der Kreatinin-Konzentration zusammengestellt.

Tabelle 1:

| Konzentration (Vol.-%) | Tyrosinase-Inhibition (%) |
|---|---|
| 0,00 | 0,00 |
| 0,05 | 1,77 |
| 0,30 | 9,97 |

Tabelle 1: (fortgesetzt)

| Konzentration (Vol.-%) | Tyrosinase-Inhibition (%) |
|---|---|
| 0,60 | 14,21 |
| 0,90 | 20,75 |
| 1,10 | 24,81 |
| 1,40 | 28,15 |
| 1,70 | 32,18 |

Die tyrosinasehemmenden Eigenschaften zeichnen Kreatinin als milden Hautaufheller aus, dessen Verwendung in Kosmetika mit depigmentierender Wirkung vorteilhaft ist.

Beispiel 2:

In-vitro-Hautmodell-Test

Der hautaufhellende Effekt wurde an einem *in-vitro*-Hautmodell, dem MelanoDerm™ der Firma MatTek validiert. Das Modell kommt in Struktur und Funktion der natürlichen Haut sehr nahe und verfügt über die in der Epidermis relevanten Zelltypen inklusive der Melanocyten als Syntheseort des Hautpigments Melanin. Weitergehende Informationen können z.B. bei M.K. King et al. In Proceedings of Society of Cosmetic Chemists Annual Scientific Meeting and Technology Showcase 1998, 35-36, gefunden werden.

Kreatinin, Kojisäuredipalmitat als Positivstandard sowie Wasser als Negativkontrolle wurden alle 48 Stunden über eine Periode von 21 Tagen auf das *in-vitro*-Hautmodell appliziert.

Ergebnis:

Das von den Melanocyten produzierte Melanin wurde quantitativ am 17. und 21. Tag detektiert. In Tabelle 2 sind die Resultate zusammengefasst.

Tabelle 2:

| | Melanin (μg) | |
|---|---|---|
| | **Tag 17** | **Tag 21** |
| Kreatinin | 94,5 | 12,67 |
| Kojisäuredipalmitat | 102,5 | 82,67 |
| Wasser | 205,0 | 162,17 |

Tabelle 2 demonstriert, dass Kreatinin bei längerer Anwendung doppelt so wirksam ist wie das als Positivstandard verwendete Kojisäuredipalmitat.

Erfindungsgemäßes Formulierungsbeispiel:

| Hautaufhellende Creme | Gew.-% |
|---|---|
| **Phase A** | |
| ABIL® Care 85 (Bis-PEG/PPG-16/16 PEG/PPG-16/16 Dimethicone; Caprylic/Capric Triglycerides) | 1,5 |
| TEGINACID® C (Ceteareth-25) | 0,5 |
| TEGIN® M(Glyceryl Stearate) | 2,0 |
| TEGO® Alkanol 1618 (Cetearyl Alcohol) | 6,0 |
| Cyclomethicone | 5,0 |
| **Phase B** | |
| Verbindung Formel (I) $R^1$, $R^2$ = H (Kreatinin) | 0,5 |

(fortgesetzt)

| Hautaufhellende Creme | Gew.-% |
|---|---|
| **Phase B** | |
| Glycerin | 3,0 |
| Wasser | ad 100 |
| **Phase C** | |
| TEGO® Carbomer 134(Carbomer) | 0,15 |
| TEGOSOFT® O (Ethylhexyl Palmitate) | 0,6 |
| **Phase D** | |
| Natriumhydroxid (10 % in Wasser) | q.s. |
| Konservierungsmittel, Parfüm | q.s. |

| **Herstellung:** | |
|---|---|
| 1. | Phase A und B auf ungefähr 75 °C erhitzen. |
| 2. | Phase A unter Rühren zu Phase B geben.[1)] |
| 3. | Homogenisieren. |
| 4. | Unter Rühren auf ca. 60 °C abkühlen und anschließend Phase C zugeben. |
| 5. | Kurz Homogenisieren. |
| 6. | Unter Rühren abkühlen und unterhalb 40 °C Phase D zugeben. |

1) **Wichtig:** Wenn Phase A vorgelegt wird, muss Phase B **ohne Rühren** zugegeben werden.

Ergebnis:

Drei Personen wandten die oben beschriebene Creme über einen Zeitraum von 2 Monaten zur Behandlung von Pigmentflecken am Oberarm zweimal täglich an. Schon nach 4 Wochen zeigte sich ein deutlich sichtbares Aufhellen und nach zwei Monaten war eine deutlich sichtbare Verbesserung des Hautbildes, d.h. eine signifikante Minderung der lokalen Pigmentstörungen festzustellen. Hautirritationen wurden während der gesamten Behandlungsdauer nicht beobachtet.

## Patentansprüche

1. Verwendung von Verbindungen der allgemeinen Formel (I) und/oder deren Salze

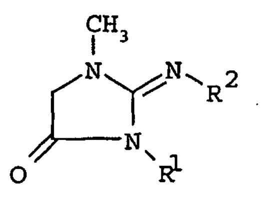

(I)

in denen

$R^1$ H, Alkyl-, Hydroxyalkyl-, Carboxyalkylrest mit 2 bis 30 C-Atomen;
$R^2$ H oder ein gegebenenfalls verzweigter, gegebenenfalls Doppelbindungen enthaltender Kohlenwasserstoffrest mit 1 bis 30 C-Atomen sein kann,

zur Aufhellung der Haut und Milderung von Pigmentstörungen

**2.** Verwendung von Verbindungen gemäß Anspruch 1, **dadurch gekennzeichnet, dass** die Reste $R^1$ und/oder $R^2$ 2 bis 20 C-Atome aufweisen.

**3.** Verwendung von Verbindungen gemäß den Ansprüchen 1 und/oder 2, **dadurch gekennzeichnet, dass** die Reste $R^1$ und/oder $R^2$ Wasserstoff sind.

**4.** Verwendung von Verbindungen gemäß mindestens einem der Ansprüche 1 bis 3, **dadurch gekennzeichnet, dass** 0,05 bis 10,0 Gew.-%, mindestens eine der Verbindungen der allgemeinen Formel (I) als Wirksubstanz enthalten ist.

**5.** Verwendung von wässrigen kosmetischen oder dermatologischen Formulierungen enthaltend

a) 0,05 bis 10 Gew.-Teile mindestens einer der Verbindungen der allgemeinen Formel (I) und

b) 1 bis 10 Gew.-Teile mindestens eines Tensids aus der Gruppe der nichtionischen, amphoteren, zwitterionischen, ionischen Tenside und gegebenenfalls

c) 2 bis 10 Gew.-Teile pflanzliche oder mineralische Öle, Esteröle

d) 1 bis 5 Gew.-Teile Konsistenzgeber

e) 0,5 bis 5,0 Gew.-Teile Duftstoffe, Farbstoffe, Pflanzenextrakte, Konservierungsmittel

f) ad 100 Wasser

zur Aufhellung der Haut und Milderung von Pigmentstörungen.

## Claims

**1.** Use of compounds of the general formula (I) and/or salts thereof

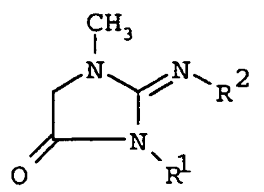

(I)

in which

$R^1$ may be H, alkyl, hydroxyalkyl, carboxyalkyl radical having 2 to 30 carbon atoms;
$R^2$ may be H or a hydrocarbon radical having 1 to 30 carbon atoms which may be branched or unbranched and may or may not contain double bonds,

for lightening the skin and toning down pigment disorders.

**2.** Use of compounds according to Claim 1, **characterized in that** the radicals $R^1$ and/or $R^2$ have 2 to 20 carbon atoms.

**3.** Use of compounds according to Claims 1 and/or 2, **characterized in that** the radicals $R^1$ and/or $R^2$ are hydrogen.

**4.** Use of compounds according to at least one of Claims 1 to 3, **characterized in that** 0.05 to 10.0% by weight of at least one of the compounds of the general formula (I) is present as active substance.

**5.** Use of aqueous cosmetic or dermatological formulations comprising

a) 0.05 to 10 parts by weight of at least one of the compounds of the general formula (I) and

b) 1 to 10 parts by weight of at least one surfactant from the group of nonionic, amphoteric, zwitterionic, ionic surfactants and optionally

c) 2 to 10 parts by weight of vegetable or mineral oils, ester oils

d) 1 to 5 parts by weight of bodying agents

e) 0.5 to 5.0 parts by weight of fragrances, dyes, plant extracts, preservatives

f) ad 100% with water

for lightening the skin and toning down pigment disorders.

## Revendications

**1.** Utilisation de composés de formule générale (I) et/ou de leurs sels

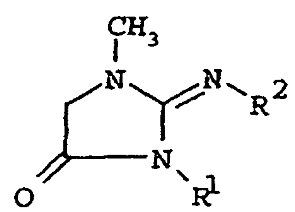

(I)

dans laquelle
$R^1$ peut représenter H, un radical alkyle, hydroxyalkyle, carboxyalkyle comprenant 2 à 30 atomes de carbone
$R^2$ peut représenter H ou un radical hydrocarboné le cas échéant ramifié, contenant le cas échéant des doubles liaisons, comprenant 1 à 30 atomes de carbone,
pour éclaircir la peau et atténuer les troubles de pigmentation.

**2.** Utilisation de composés selon la revendication 1, **caractérisée en ce que** les radicaux $R^1$ et/ou $R^2$ présentent 2 à 20 atomes de carbone.

**3.** Utilisation de composés selon les revendications 1 et/ou 2, **caractérisée en ce que** les radicaux $R^1$ et/ou $R^2$ représentent hydrogène.

**4.** Utilisation de composés selon au moins l'une quelconque des revendications 1 à 3, **caractérisée en ce que** 0,05 à 10,0% en poids d'au moins un des composés de formule générale (I) est contenu comme substance active.

**5.** Utilisation de formulations aqueuses cosmétiques ou dermatologiques, contenant

a) 0,05 à 10 parties en poids d'au moins un des composés de formule générale (I) et
b) 1 à 10 parties en poids d'au moins un tensioactif du groupe des agents tensioactifs non ioniques, amphotères, zwittérioniques, ioniques, et le cas échéant
c) 2 à 10 parties en poids d'huiles végétales ou minérales, d'huiles d'ester,
d) 1 à 5 parties en poids d'un agent conférant une consistance,
e) 0,5 à 5,0 parties en poids de parfums, de colorants, d'extraits végétaux, de conservateurs,
f) jusqu'à 100 d'eau

pour éclaircir la peau et atténuer les troubles de la pigmentation.